# EUROPÄISCHE PATENTSCHRIFT

(11) **EP 1 894 512 B1**
(45) Veröffentlichungstag und Bekanntmachung des Hinweises auf die Patenterteilung: **01.12.2010**
(21) Anmeldenummer: 07113127.0
(22) Anmeldetag: 25.07.2007
(51) Int. Cl.: A61B 1/00, A61B 1/01, A61B 1/31

(54) **Stülpschlauch mit 2-Komponentenschmiermittel**
Flexible tube with 2-component lubricant
Tube à retournement à additif de lubrifiant à 2 composants

(30) Priorität: 01.09.2006 DE 102006000435
(43) Veröffentlichungstag der Anmeldung: 05.03.2008
(73) Patentinhaber: INVENDO MEDICAL GMBH, 69469 Weinheim (DE)
(72) Erfinder: Bob, Konstantin, 69469, Weinheim (DE); Pauker, Fritz, 86438, Kissing (DE); Viebach, Thomas, 82282 Pischertshofen (DE)
(74) Vertreter: Winter, Brandl, Fürniss, Hübner, Röss, Kaiser, Polte - Partnerschaft

(56) Entgegenhaltungen:
- EP-A- 1 607 038
- WO-A-92/00036
- US-A- 4 876 126
- US-A- 5 242 428
- US-A1- 2003 229 269

## Beschreibung

Die vorliegende Erfindung bezieht sich auf eine medizintechnische Vorrichtung, die mit einem menschlichen oder tierischen Körper in Eingriff gebracht werden kann, und genauer gesagt auf ein Endoskop.

Endoskopievorrichtungen sowie Vorrichtungen zum Einführen eines medizinischen Endoskops in einen Körperkanal werden beispielsweise in der offengelegten deutschen Patentanmeldung DE-A-39 25 484 oder WO 92/00036 beschrieben. Die darin beschriebenen Vorrichtungen erlauben es, dass ein Endoskop nicht mehr in den zu untersuchenden Körper hineingeschoben wird, sondern sich selber hineinbewegt. Zu diesem Zweck ist das Endoskop mit einem Eigenantrieb ausgestattet, der ein unkomplizierteres und schnelleres Einführen ermöglicht.

Als solch ein Eigenantrieb kann beispielsweise auch ein so genannter Stülpschlauch verwendet werden, in den der Endoskopschaft eingeführt ist. Beim Vortrieb des Endoskops treten unterschiedliche Relativbewegungen auf. Zum einen tritt eine Relativbewegung zwischen dem Endoskopschaft und dem Stülpschlauch auf, die miteinander in Gleitkontakt stehen. Zum anderen tritt auch eine Relativbewegung zwischen einem innenliegenden und einem außenliegenden Abschnitt des sich abwickelnden Stülpschlauchs auf.

Um die jeweils auftretende Gleitreibung sowie eine Haftreibung zwischen jeweiligen Elementen zu verringern, ist beispielsweise der Einsatz eines von Außen zugeführten Schmiermittels vorgeschlagen worden, beispielsweise in der EP-A-0 873 761. In beiden Fällen wird zwischen den Endoskopschaft und den Stülpschlauch einerseits sowie zwischen die beiden übereinander zu liegen kommenden Stülpschlauchabschnitte andererseits ein Schmiermittel zugeführt. Die zum Einsatz kommenden Schmiermittel sind zumeist Öle, wie z.B. Speiseöle. Bei Ölen besteht jedoch das Problem, dass diese z.B. entlang des Endoskopschafts wandern können und die Linse des Endoskops mit einem Schmierfilm bedecken können, welcher nicht leicht entfernbar ist.

Demzufolge besteht die Aufgabe der vorliegenden Erfindung in der Bereitstellung einer verbesserten medizintechnischen Vorrichtung, insbesondere einer verbesserten Endoskopievorrichtung. Spezielle Verbesserungen, die vorzugsweise erzielt werden sollen, sind insbesondere eine Vereinfachung des Aufbaus der medizintechnischen Vorrichtung, erhöhte Wirtschaftlichkeit, verbesserte Akzeptanz beim Individuum, bei dem die Vorrichtung zum Einsatz kommt, sowie eine Verringerung der Reibungskräfte, die in der medizintechnischen Vorrichtung auftreten.

Erfindungsgemäß wird die Aufgabe mit einer medizintechnischen Vorrichtung gemäß Anspruch 1 gelöst.

Weitere vorteilhafte Ausgestaltungen der Erfindung sind Gegenstand der Unteransprüche.

Demgemäß besteht der Kern der Erfindung darin, dass eine medizintechnische Vorrichtung, wie z.B. ein Endoskop, mindestens ein Element hat, welches mit einer ersten Komponente versehen ist, die erst unter Zufuhr einer zweiten Komponente schmierende Eigenschaften entfaltet. D.h. ein im Betrieb der medizintechnischen Vorrichtung reibungsausgesetztes Element, wie z.B. ein Stülpschlauch oder ein Endoskopschaft/Endoskopschafthülle eines Endoskops, wird vorab mit einer ersten Komponente in Festform, wie z.B. Cellulose beschichtet, welche bei Inbetriebnahme der medizintechnischen Vorrichtung bzw. des Endoskops mit einer zweiten Komponente wie z.B. Wasser verflüssigt wird, um dadurch schmierende Eigenschaften zu entfalten. Somit wird eine Reibung zischen dem im Betrieb reibungsausgesetzten Element und mit diesem Element sich in Reibkontakt befindlichen Elementen verringert.

Bevorzugt ist die medizintechnische Vorrichtung ein Endoskop.

Vorteilhafterweise ist das Element ein Abschnitt bzw. ein Bestandteil der medizintechnischen Vorrichtung, der/das im Betrieb der medizintechnischen Vorrichtung starker Reibung ausgesetzt ist. Vorzugsweise ist das Element ein Stülpschlauch und/oder ein Endoskopschaft/Endoskopschafthülle eines Endoskops. Dadurch kann eine Haftreibung und Gleitreibung zwischen einem Stülpschlauch und einem Endoskopschaft/einer Endoskopschafthülle des Endoskops und/oder zwischen den sich relativ bewegenden Flächen des Stülpschlauchs verringert werden. Das Element kann aber auch jedes andere Bestandteil bzw. ein Abschnitt eines Bestandteils der medizintechnischen Vorrichtung sein, das/der im Betrieb der endoskopischen Vorrichtung einer Reibung ausgesetzt ist.

Im allgemeinen ist die erste Komponente wenigstens an einem Teil einer Außenfläche und/oder einer Innenfläche des Elements vorgesehen. Alternativ kann aber auch die gesamte Fläche des Elements (sowohl Außenfläche als auch Innenfläche) mit der ersten Komponente versehen sein. Bevorzugt ist sowohl die gesamte Innen- als auch Außenfläche des Stülpschlauchs mit der ersten Komponente versehen.

Bevorzugt hat die erste Komponente vor einer Zufuhr mit der zweiten Komponente eine hohe Bindungsenergie bezüglich des Elements. Vorteilhafterweise ist die Bindungsenergie so hoch, dass ein Ablösen der ersten Komponente von dem Element, insbesondere an Stellen mit starker Verformung des Elements, wie z.B. den Umstülpstellen eines Stülpschlauchs, wirksam verhindert wird. In diesem Zusammenhang ist es auch wünschenswert, dass die erste Komponente vor einer Zufuhr mit der zweiten Komponente außerdem eine hohe Biegeelastizität aufweist.

Auch ist erwünscht, dass die erste Komponente vor Zufuhr der zweiten Komponente möglichst in Festform vorliegt, so dass ein mögliches Austrocknen, z.B. bei langer Lagerung der medizintechnischen Vorrichtung bzw. des Elements, nicht berücksichtigt werden muss. Dies ist aber keine notwendige Voraussetzung für den Gegenstand der Erfindung.

Vorteilhafterweise ist die erste Komponente in Kavitäten untergebracht, die in dem Stülpschlauch ausgebildet sind.

Bevorzugt werden die Kavitäten durch eine Plasmabehandlung ausgebildet.

Vorteilhafterweise ist des Element der medizintechnischen Vorrichtung aus Kautschuk, bevorzugt aus Silikonkautschuk, ausgebildet.

Vorteilhafterweise ist die erste und/oder zweite Komponente aus einem körperverträglichen oder bioabbaubarem Material ausgebildet.

Vorteilhafterweise werden als die erste Komponente quellfähige Makromoleküle verwendet. Bevorzugt ist die erste Komponente ein Polysaccharid. Vorteilhafterweise besteht die erste Komponente in diesem Fall aus Stärke, Glykogen, Cellulose, rekombinanter Cellulose oder Agar-Agar. Bevorzugt besteht die erste Komponente aus einem Alginat mit Soja-Proteinzusatz und/oder Molke-Proteinzusatz.

Alternativ kann die erste Komponente ein Peptid sein. In diesem Fall besteht die erste Komponente vorteilhafterweise aus Kollagen, Gelatine oder rekombinanter Gelatine.

Des weiteren kann die erste Komponente auch ein Kunststoff oder ein Silikat sein.

Bevorzugt ist die zweite Komponente Öl, Wasser, oder eine Öl-Wasser-Emulsion.

Alternativ könnte aber auch Körperflüssigkeit, wie z.B. im Darm enthaltene Flüssigkeit als die zweite Komponente verwendet werden.

Des weiteren ist es denkbar, dass die zweite Komponente aus Alkoholen besteht, wie z.B. Ethanol, Glukol, Propanol oder Glycerin.

Bevorzugt ist die medizintechnische Vorrichtung ein Endoskop mit einem Endoskopschaft, der evtl. eine Endoskopschafthülle aufweist, und einem Stülpschlauch, der den Endoskopschaft zumindest teilweise umgibt.

Vorteilhafterweise wird die zweite Komponente an Stellen der medizintechnischen Vorrichtung aufgebracht, die im Betrieb der medizintechnischen Vorrichtung starker Reibung ausgesetzt sind. Bevorzugt wird bei dem Endoskop die zweite Komponente an einer vorderen Umstülpung und/oder an einer hinteren Umstülpung des Stülpschlauchs aufgebracht wird. Die zweite Komponente kann aber auch zusätzlich oder alternativ zwischen dem Endoskopschaft und dem Stülpschlauch aufgebracht werden. Es ist aber auch möglich, die zweite Komponente zusätzlich oder alternativ zwischen umgestülpten Flächen des Stülpschlauchs aufzubringen und/oder auf einer dem Endoskopschaft abgewandten Fläche des Stülpschlauchs aufzubringen.

Bevorzugt wird die zweite Komponente zu der ersten Komponente durch einen in dem Endoskopschaft ausgebildeten Kanal zugeführt. Dieser Kanal kann z.B. ein Arbeitskanal, ein Kühlmittelkanal oder ein anderer in dem Endoskop ausgebildeter Kanal sein. Die zweite Komponente muss aber nicht zwangsläufig durch einen in dem Endoskop ausgebildeten Kanal zugeführt werden, sondern kann z.B. durch einen extern ausgebildeten Kanal zugeführt werden.

Vorteilhafterweise weist das Endoskop des weiteren eine externe Versorgungskammer auf, in welcher die zweite Komponente gespeichert und von welcher die zweite Komponente zugeführt wird.

Die Erfindung wird nachfolgend anhand eines bevorzugten Ausführungsbeispiels unter Bezugnahme auf die begleitende Zeichnung näher erläutert.

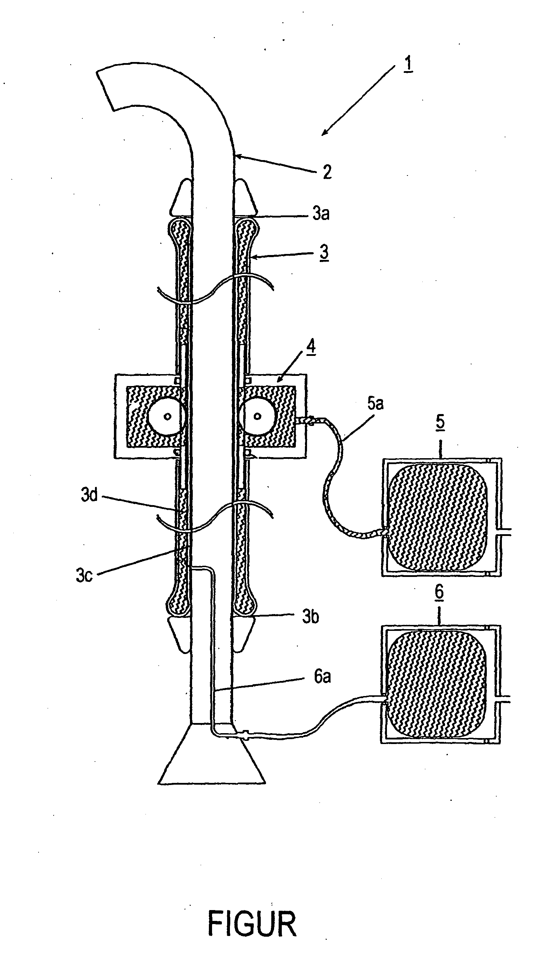

Fig. 1 zeigt schematisch den Aufbau eines Endoskops mit einem Stülpschlauch gemäß einer bevorzugten Ausführungsform der vorliegenden Erfindung. Obwohl in dieser Ausführungsform nur der Stülpschlauch mit der ersten Komponente versehen ist, die erst bei Aktivierung mit einer zweiten Komponente ihre schmierenden Eigenschaften entfaltet, sollte klar sein, dass andere bzw. zusätzliche Elemente des Endoskops mit der ersten Komponente versehen sein können.

Wie in Fig. 1 gezeigt ist, besteht das Endoskop 1 im Wesentlichen aus einem Endoskopschaft 2, einem Stülpschlauch 3 und aus einer Antriebsvorrichtung 4. Zusätzlich sind in diesem Ausführungsbeispiel zwei externe Reservoirkammern 5 und 6 vorgesehen, welche jeweils mit Leitungen 5a und 6a mit einer Innenseite 3d bzw. Außenseite 3a, 3b, 3d des Stülpschlauchs 3 in Fluidverbindung stehen.

Erfindungsgemäß ist der Stülpschlauch 3 mit einer ersten Komponente versehen, welche erst bei Aktivierung durch bzw. Zusammenwirken mit einer zweiten Komponente schmierende Eigenschaften aufweist, d.h. das Schmiermittel selbst wird erst durch Aktivierung der ersten Komponente durch die zweite Komponente gebildet.

Dadurch kann der Stülpschlauch 3 schon bei der Herstellung mit der ersten Komponente, dem eigentlichen Schmiermittel, versehen werden, ohne dass dieses seine schmierenden Eigenschaften verlieren könnte, da es diese erst mit der Aktivierung durch die zweite Komponente an den Tag legt. Im Gegensatz zu Stülpschläuchen, die vor einer eigentlichen Verwendung mit Öl beschichtet sind, besteht der Vorteil, dass während einer Lagerung bzw. vor einer eigentlichen Verwendung des Stülpschlauchs, der Bestandteil einer medizinischen Vorrichtung ist, kein Schmiermittel von dem Stülpschlauch zu anderen Teilen der medizintechnischen Vorrichtung austreten bzw. wandern kann.

Vorzugsweise hat die erste Komponente vor einer Aktivierung mit der zweiten Komponente eine Festform, deren Eigenschaften auch bei langer Lagerung nicht beeinträchtigt werden.

Die erste Komponente ist des weiteren bevorzugt über sowohl die gesamte Innenfläche 3d als auch die gesamte Außenfläche 3c des Stülpschlauchs aufgebracht. Vorzugsweise ist die erste Komponente homogen aufgebracht.

Das Aufbringen der ersten Komponente auf den Stülpschlauch 3 kann durch jedes beliebige, geeignete herkömmlichen Verfahren realisiert werden, wie z.B. Tauchbeschichtung, Walzenüberziehen, Rakelüberziehen, Rotationsbeschichten (Spin Coating), das so genannte Doctor-Blade-Verfahren usw. Vorteilhafterweise wird mit dem verwendeten Verfahren eine gleichmäßiges und damit besonders glattes Aufbringen der ersten Komponente erzielt.

Der Stülpschlauch 3 unterliegt besonders an seinen Umstülpstellen starker Verformung. Deshalb weist die erste die erste Komponente vorteilhaft eine hohe Bindungsenergie bezüglich des Stülpschlauchs 3 sowie eine hohe Biegeelastizität auf, um zu gewährleisten, dass sich die erste Komponente nicht von dem Stülpschlauch 3 ablöst. Auch im Hinblick auf die Aktivierung ist es wünschenswert, dass die erste Komponente nach einer Aktivierung durch die zweite Komponente eine ausreichend hohe Bindungsenergie bezüglich des Stülpschlauchs hat, damit das auf diese Weise ausgebildete Schmiermittel an den dafür vorgesehen Stellen verbleibt und nicht bzw. möglichst nur in geringen Mengen in den untersuchten menschlichen oder tierischen Körper gelangt. Um die Bindungsenergie der ersten Komponente bezüglich des Stülpschlauchs 3 zu erhöhen, ist es z.B. denkbar, die Oberfläche des Stülpschlauchs anzurauhen oder mit Nuten zu versehen, um ein besseres Anhaften der ersten Komponente zu ermöglichen. In diesem Zusammenhang ist auch eine chemische Vorbehandlung denkbar.

Um eine besonders gute Absonderung des ausgebildeten Schmiermittels sowie eine Bevorratung der ersten Komponente direkt an der Oberfläche des Stülpschlauchs 3 zu gewährleisten, ist es bevorzugt, dass wenigstens eine Oberfläche des Stülpschlauchs 3 ein Profil aufweist, dessen Kavitäten die erste Komponente beinhalten. Die Kavitäten, z.B. in der Form von Nuten, können durch Prägen, Aufschäumen und Aufschneiden, Schneiden oder Walzen oder durch eine Gravur, insbesondere eine Lasergravur, erzeugbar sein.

In einer bevorzugten Ausgestaltung die kein Teil der Erfindung ist, befindet sich die erste Komponente daher in Nuten, die direkt an einer Oberfläche des Stülpschlauchs 3 angeordnet sind. In einigen Ausgestaltungen bilden regelmäßig angeordnete Nuten, in anderen Ausgestaltungen unregelmäßig angeordnete Nuten das Profil.

Alternativ kann der Stülpschlauch eine Schichtstruktur aufweisen. Die Schichtstruktur umfasst mindestens eine Substratschicht, um eine stabile Grundlage für darauf angeordnete Schichten auszubilden, eine auf mindestens einer, vorzugsweise beiden Seiten des Substrats angeordnete Reservoirschicht für die erste Komponente, um genügend Schmiermittel für den tatsächlichen Einsatz zu bevorraten, und mindestens eine bzw., wenn auf beiden Seiten des Substrats eine Reservoirschicht angeordnet ist, zwei außenliegende perforierte Schicht(en), um das Schmiermittel, d.h. die erste Komponente nach Aktivierung durch die zweite Komponente, in kontrollierter Weise abzugeben. Im ersten Fall ergibt sich der grundsätzliche Aufbau Substrat/Reservoirschicht/perforierte Schicht, im zweiten Fall der grundsätzliche Aufbau perforierte Schicht/Reservoirschicht/Substrat/Reservoirschicht/perforierte Schicht, wobei jeweils noch Zwischenschichten vorgesehen sein können.

Die Substratschicht wird dabei durch den Stülpschlauch 3 selbst gebildet, wobei dieser vorteilhaft aus Siliconkautschuk ausgebildet ist.
Die Reservoirschicht ist eine Schicht, die aus der ersten Komponente besteht, die bevorzugt in Festform vorliegt. Durch Aktivierung dieser Schicht mit der zweiten Komponente wird die erste Komponente verflüssigt bzw. quillt auf, d.h. sie entfaltet ihre schmierende Eigenschaften, und das auf diese Weise entstehende Schmiermittel wird anschließend an mindestens eine benachbarte Schicht abgegeben.

Es ist auch möglich, dass die Reservoirschicht mit einer schwammartigen Struktur ausgebildet oder durch ein Material, welches Kapillarwirkung entfaltet, gebildet ist, wobei die erste Komponente von der schwammartigen Struktur bzw. durch das Material aufgesogen ist und wiederum durch Aktivierung durch die zweite Komponente an mindestens eine benachbarte Schicht abgegeben wird.

Im dem Fall, dass zwei Reservoirschichten vorliegen, können diese gleich oder unterschiedlich ausgebildet sein. Eine unterschiedliche Ausgestaltung kann insbesondere vorteilhaft sein, wenn unterschiedliche Druckeinflüsse auf den Stülpschlauch 3 vorliegen. Bei dem Stülpschlauch 3 treten insbesondere im Umstülpbereich unterschiedliche physikalische Einflüsse wie z.B. Druck bei der innenliegenden und der außenliegenden Reservoirschicht auf, so dass hier eine unterschiedliche Ausgestaltung dieser Schicht, die jeweils den äußeren Bedingungen angepasst ist, nützlich sein kann. Dieselbe Überlegung gilt auch für die hier nachstehend beschriebene perforierte Schicht.

Vorzugsweise ist die perforierte Schicht direkt angrenzend an die Reservoirschicht bereitgestellt und bildet die äußere Oberfläche des Elements aus. Allerdings können zwischen der Reservoirschicht und der perforierten Schicht weitere Schichten angeordnet sein, solange sie für die erste Komponente bzw. das durch Aktivierung mit der zweiten Komponente gebildete Schmiermittel im Wesentlichen durchlässig sind. Die Perforation der perforierten Schicht kann jede beliebige Gestalt aufweisen. Es ist bevorzugt, dass diese Schicht durchgehende Löcher bzw. Kanäle aufweist, deren Mittelachsen mit der Oberfläche des Elements, dessen Oberfläche durch die perforierte Schicht gebildet wird, einen nahezu rechten Winkel einschließen, also von z.B. 80 bis 100°, insbesondere von etwa 90°. Auf diese Weise gelangt das ausgebildete Schmiermittel in besonders effektiver und kontrollierter Weise von der Reservoirschicht an die zu schmierende Oberfläche.

Das schichtweise aufgebaute Element erlaubt eine einfache Herstellung, indem z.B. die einzelnen Schichten wie erforderlich vorausgehend hergestellt und dann zusammenlaminiert werden oder indem die Schmiermittelschicht auf das Substrat (Stülpschlauch 3) bzw. die oberste von ggf. über dem Substrat angeordneten Schichten aufgebracht und dieser Verbund dann mit einer perforierten Schicht versehen wird. Ein Vorteil dieser Ausgestaltung ist, dass das Schmiermittelreservoir bzw. das Reservoir für die erste Komponente mit beliebigen, anwendungsabhängigen Größen ausgestaltet werden kann.

Das vorstehend erwähnte Profil ist vorzugsweise so angepasst, dass freigesetztes Schmiermittel zu den einer besonders großen Reibung ausgesetzten Bereichen zugeführt wird. Hierzu kann das Profil längsverlaufende, querverlaufende oder spiralförmig verlaufende Kavitäten aufweisen, die vorzugsweise durch Nuten bzw. Rillen oder durch einzelne, lokal begrenzte Vertiefungen gebildet werden.

Ein zum Zweck der Aufnahme der ersten Komponente und kontrollierten Schmiermittelabgabe geeignetes Profil lässt sich durch Gravieren, insbesondere durch Lasergravieren (auch als Laserengraving bekannt) erzeugen. Ein Stülpschlauch 3, der ein durch Lasergravieren erzeugbares Profil aufweist, ist nicht in die vorliegende Erfindung eingeschlossen.

Das Lasergravieren erlaubt einen kontrollierten Materialabtrag, der z.B. mittels eines gepulsten Laserstrahls stattfindet. Der Strahl wird dabei vorzugsweise zur Verbesserung der Bearbeitungsgenauigkeit sehr stark fokussiert, wobei z.B. Brennpunkte mit einem Durchmesser 0,01 bis 0,1 mm erzeugt werden. Im Brennpunkt können sehr hohe Temperaturen von beispielsweise größer 2000°C erreicht werden, was eine schnelle Bearbeitung nahezu beliebiger Materialien ermöglicht.

Bei einem Gravierungsschritt können, seitlich von den erzeugten Kavitäten, Aufwerfungen auf der behandelten Oberfläche auftreten. Derartige Aufwerfungen verringern die Gleiteigenschaften des Stülpschlauchs 3. Daher ist es weitergehend bevorzugt, dass das Profil durch Gravieren mit einem Laser und ein anschließendes Glätten erhältlich ist.

Als besonders geeignete Methoden zum Glätten der gravierten Oberfläche, so dass die Aufwerfungen nivelliert werden und gleichzeitig eine zur Aufnahme einer ausreichenden Menge der erste Komponente geeignete Tiefe der eingravierten Kavitäten verbleibt, haben sich Keramikbeschichtung, Silicatisierung, UV-Bestrahlung, Acrylatbeschichtung und Polysilazanbeschichtung erwiesen.

Es ist zudem möglich, eine der vorstehenden Methoden zur Oberflächenbehandlung ohne vorausgehendes Lasergravieren einzusetzen. In diesem Fall wird eine Schicht der ersten Komponente auf der Oberfläche erzeugt.

Erfindungsgemäß für das Ausbilden von Kavitäten in dem Stülpschlauch zur Aufnahme der ersten Komponente ist der Einsatz einer herkömmlich bekannten Plasmabehandlung, wie z.B. vom Fraunhofer Institut für Grenzflächen und Verfahrenstechnik eingesetzt. Ein derartiges Plasmaverfahren dient in seiner bekannten Anwendung dazu, die Oberfläche eines Substrats anzurauen bzw. zu aktivieren, um die Haftungseigenschaften des Substrats zu erhöhen. Die Anmelderin hat jedoch herausgefunden, dass die Kavitäten, die durch das Plasmaverfahren ausgebildet werden, ausreichend sind, um die erste Komponente (bevorzugt ein Alginat) einbringen zu können, und das die dadurch erreichbare Schmierwirkung ausreichend für eine zufriedenstellende Schmierung des Stülpschlauchs bzw. Endoskopschafts/Endoskopschafthülle ist. Daher wird das herkömmlich bekannte Plasmaverfahren über seinen ursprünglichen Zweck hinaus zur Erzeugung von Kavitäten verwendet, in denen die erste Komponenten aufgenommen wird. In diesem Zusammenhang ist die Verwendung eines Alginats als erste Komponente bevorzugt.

Da der Stülpschlauch 3 vorzugsweise für ein Endoskop verwendet wird, ist es vorteilhaft, dass die erste Komponente und zweite Komponente körperverträglich sind, bzw. derart beschaffen sind, dass sie in den verwendeten Mengen für den zu untersuchenden Körper unschädlich sind. Die erste und zweite Komponente können auch aus bioabbaubaren Materialien bestehen.

Erfindungsgemäß entfaltet die erste Komponente ihre schmierende Komponente bzw. quillt die erste Komponente auf, erst nach einer Aktivierung durch eine zweite Komponente.

Als erste Komponente ist die Verwendung von Polysacchariden, wie Stärke, Glykogen, Cellulose, rekombinante Cellulose (wird z.B. von Mikroorganismen oder transgenen Pflanzen gewonnen), Agar-Agar oder ein Alginat mit Soja-Proteinzusatz und/oder Stärke-Proteinzusatz. besonders bevorzugt. Diese Elemente können sehr einfach in Festform als Schicht ausgebildet werden, und sind zudem besonders körperverträglich. Alternativ sind auch Peptide wie z.B. Kollagen, Gelatine oder rekombinante Gelatine (wird z.B. von Mikroorganismen oder transgenen Pflanzen gewonnen) für eine Verwendung als erste Komponente hervorragend geeignet, da auch sie besonders leicht als Schicht auszubilden sind, sowie eine besonders gute Körperverträglichkeit aufweisen. Des weiteren können auch andere quellbare Makromoleküle oder auch Kunststoffe für eine derartige Verwendung in Betracht gezogen werden.

Bei Verwendung der vorstehend beschrieben Elemente als erste Komponente ist die Verwendung von Wasser als zweite Komponente besonders vorteilhaft, da dieses allgemein verfügbar und körperverträglich ist. Durch Aktivierung mit Wasser verflüssigen die vorstehend genannten Elemente bzw. quellen auf, und entfalten somit ihre schmierenden Eigenschaften. Es ist aber auch denkbar, z.B. bei Verwendung des Stülpschlauchs in einer endoskopischen Vorrichtung, Körperflüssigkeit, die in dem zu untersuchenden Bereich vorhanden ist, als die zweite Komponente zu verwenden. Für eine Verwendung als zweite Komponente sind auch Alkohole wie z.B. Ethanol, Glykol, Propanol oder Glycerin gut geeignet.

Bei Verwendung der vorstehenden ersten und zweiten Komponenten zur Ausbildung eines Schmiermittels besteht im Gegensatz zu Ölen, die, falls sie z.B. zu einer Linse eines Endoskops gelangen, auf dieser einen fetten, dickflüssigen, schwer ablösbaren Schmierfilm ausbilden, der Vorteil, dass, das Schmiermittel sehr leicht von der Linse entfernt werden kann, z.B. durch einen schwachen Wasserstrahl.

Mit Bezug auf Fig. 1, wird die zweite Komponente in zwei Reservoirs (5, 6) gespeichert, von denen aus die zweite Komponente für eine Aktivierung der ersten Komponente jeweils über Leitungen 5a und 6a zu dem Stülpschlauch befördert wird, an dem die erste Komponente vorgesehen ist. Die Leitung 5a ist mit einem Gehäuse des Antriebs 4 verbunden, um die zweite Komponente auf die Innenfläche 3d des Stülpschlauchs 3 aufzubringen. Die Leitung 6a führt durch den Endoskopschaft 2 und öffnet an einer Außenseite des Endoskopschafts 2 gegenüber einer Außenseite 3c des Stülpschlauchs 3. Somit werden sowohl die Innenseite 3d als auch die Außenseite 3a, 3b, 3c des Stülpschlauchs mit der zweiten Komponente beaufschlagt.

Die Erfindung ist nicht auf die vorstehend beschriebene Anordnung beschränkt, sondern kann vielfältig abgewandelt werden. Wichtig ist vor allem, dass die zweite Komponente sicher zu einem Bereich des Elements/Stülpschlauchs zugeführt wird, an dem die erste Komponente aufgebracht ist. Demgemäß kann z.B. nur ein gemeinsames Reservoir ausgebildet sein, von dem zwei Leitungen abzweigen, oder, z.B. bei der Verwendung von Körperflüssigkeit, kann auf Reservoire und/oder Leitungen gänzlich verzichtet werden. Es ist aber auch denkbar, dass die zweite Komponente nur über eine Leitung zugeführt wird, die sich entweder durch den Endoskopschaft erstreckt oder von dem Endoskopschaft getrennt ausgebildet ist. Im Fall des Verwendens einer Leitung, die sich durch den Endoskopschaft erstreckt wird, muss diese nicht unbedingt neu geschaffen werden, sondern es können bestehende Leitungen oder Kanäle, wie z.B. Arbeitskanal, Kühlkanal oder ein Kanal für eine Sprühdüsenversorgung, teilweise oder vollständig genutzt werden.

In dem Ausführungsbeispiel, das in Figur 1 gezeigt ist, wird die zweite Komponente an der Innenseite 3d des Stülpschlauchs 3 und an bei einer Stelle an der Außenseite 3c des Stülpschlauchs zwischen Stülpschlauch 3 und Endoskopschaft 2 aufgebracht. Die Erfindung ist jedoch nicht auf eine derartige Anordnung beschränkt. Bevorzugt wird die zweite Komponente an Bereichen auf dem Stülpschlauch aufgebracht, die besonders hoher Reibung ausgesetzt sind. So ist es denkbar, dass die zweite Komponente z.B. an einer oder beiden Umstülpstellen 3a, 3b des Stülpschlauchs aufgebracht wird.

Eine medizintechnische Vorrichtung, wie ein Endoskop, weist einen Stülpschlauch auf, der einen Endoskopschaft für ein Antreiben von diesem umgibt. Um eine Reibung aufgrund einer Relativbewegung zwischen dem Endoskopschaft und dem Stülpschlauch und/oder eine Reibung zwischen zwei gegenüberliegenden Flächen des Stülpschlauchs zu minimieren, werden diese Bereiche mit einem Schmiermittel beaufschlagt. Dazu wird der Stülpschlauch und/oder der Endoskopschaft mit einer ersten Komponente beschichtet, die in Festform vorliegt. Durch Zufuhr einer zweiten Komponente zu der ersten Komponente, die mit der ersten Komponente reagiert, verflüssigt sich die erste Komponente und/oder quillt auf, um ihre schmierenden Eigenschaften zu entfalten.

## Patentansprüche

1. Medizintechnische Vorrichtung (1) mit mindestens einem Element (2, 3), das mit einer ersten Komponente versehen ist, welche erst unter Zuführung einer zweiten Komponenten schmierende Eigenschaften entfaltet,
**dadurch gekennzeichnet, dass**
die erste Komponente in Kavitäten untergebracht ist, die in der Oberfläche des Elements (2, 3) ausgebildet sind, und
die Kavitäten durch Plasmabehandlung erzeugt sind.

2. Medizintechnische Vorrichtung gemäß Anspruch 1, wobei das Element (2, 3) ein Stülpschlauch (3) und/oder ein Endoskopschaft (2) und/oder eine Endoskopschafthülle ist.

3. Medizintechnische Vorrichtung gemäß Anspruch 1 oder 2, **dadurch gekennzeichnet, dass** die erste Komponente vor einer Zufuhr mit der zweiten Komponente in Festform vorliegt.

4. Medizintechnische Vorrichtung gemäß einem der Ansprüche 1 bis 3,
**dadurch gekennzeichnet, dass** die erste Komponente wenigstens teilweise eine Außen- und/oder Innenfläche des Elements (2, 3) bedeckt.

5. Medizintechnische Vorrichtung gemäß einem der Ansprüche 1 bis 4,
**dadurch gekennzeichnet, dass** die erste Komponente eine hohe Bindungsenergie bezüglich des Elements hat.

6. Medizintechnische Vorrichtung gemäß einem der Ansprüche 1 bis 5,
**dadurch gekennzeichnet, dass** die erste Komponente eine hohe Biegeelastizität aufweist.

7. Medizintechnische Vorrichtung gemäß einem der Ansprüche 1 bis 6,
**dadurch gekennzeichnet, dass** das Element (3) aus Silikonkautschuk ausgebildet ist.

8. Medizintechnische Vorrichtung gemäß einem der Ansprüche 1 bis 7,
**dadurch gekennzeichnet, dass** die erste und zweite Komponente aus einem körperverträglichen und/oder bioabbaubaren Material ausgebildet sind.

9. Medizintechnische Vorrichtung gemäß einem der Ansprüche 1 bis 8,
**dadurch gekennzeichnet, dass** die erste Komponente ein Polysaccharid ist.

10. Medizintechnische Vorrichtung gemäß einem der Ansprüche 1 bis 8, dass die erste Komponente aus Stärke, Glykogen, Cellulose, rekombinanter Cellulose, Agar-Agar oder einem Alginat mit Soja-Protein-Zusatz und/oder Molke-Protein-Zusatz besteht.

11. Medizintechnische Vorrichtung gemäß einem der Ansprüche 1 bis 8,
**dadurch gekennzeichnet, dass** die erste Komponente ein Peptid ist.

12. Medizintechnische Vorrichtung gemäß Anspruch 11, **dadurch gekennzeichnet, dass** die erste Komponente aus Kollagen, Gelatine oder rekombinanter Gelatine besteht.

13. Medizintechnische Vorrichtung gemäß einem der Ansprüche 1 bis 8,
**dadurch gekennzeichnet, dass** die erste Komponente ein Kunststoff ist.

14. Medizintechnische Vorrichtung gemäß einem der Ansprüche 1 bis 13,
**dadurch gekennzeichnet, dass** die zweite Komponente Öl, Wasser, eine Öl-Wasser-Emulsion oder Körperflüssigkeit ist.

15. Medizintechnische Vorrichtung gemäß einem der Ansprüche 1 bis 13,
**dadurch gekennzeichnet, dass** die zweite Komponente Ethanol, Glykol, Propanol oder Glycerin ist.

16. Medizintechnische Vorrichtung gemäß einem der Ansprüche 1 bis 15,
**dadurch gekennzeichnet, dass** die medizintechnische Vorrichtung ein Endoskop (1) mit einem Endoskopschaft (2) und einem den Ensoskopschaft (2) zumindest teilweise umgebenden Stülpschlauch (3) ist.

17. Medizintechnische Vorrichtung gemäß Anspruch 16, **dadurch gekennzeichnet, dass** die zweite Komponente an einer vorderen Umstülpung (3a) und/oder an einer hinteren Umstülpung (3b) des Stülpschlauchs (3) aufgebracht wird.

18. Medizintechnische Vorrichtung gemäß einem der Ansprüche 16 oder 17,
**dadurch gekennzeichnet, dass** die zweite Komponente zwischen dem Endoskopschaft (2) und dem Stülpschlauch (3) aufgebracht wird.

19. Medizintechnische Vorrichtung gemäß einem der Ansprüche 16 bis 18,
**dadurch gekennzeichnet, dass** die zweite Komponente zwischen umgestülpten Flächen des Stülpschlauchs (3) aufgebracht wird.

20. Medizintechnische Vorrichtung gemäß einem der Ansprüche 16 bis 19,
**dadurch gekennzeichnet, dass** die zweite Komponente auf einer dem Endoskopschaft (2) abgewandten Fläche des Stülpschlauchs aufgebracht wird.

21. Medizintechnische Vorrichtung gemäß einem der Ansprüche 16 bis 20,
**dadurch gekennzeichnet, dass** die zweite Komponente durch einen in dem Endoskopschaft (2) ausgebildeten Kanal (6a) zugeführt wird.

22. Medizintechnische Vorrichtung gemäß einem der Ansprüche 16 bis 21,
**dadurch gekennzeichnet, das** des weiteren eine externe Versorgungskammer (5, 6) aufweist, in welcher die zweite Komponente gespeichert und von welcher die zweite Komponenten zugeführt wird.

23. Verfahren zum Versehen von mindestens einem Element (2, 3) einer medizintechnischen Vorrichtung (1) mit einer ersten Komponente, welche erst durch Aktivierung mittels einer zweiten Komponente seine schmierenden Eigenschaften entfaltet,
**gekennzeichnet durch**
Erzeugen von Kavitäten auf der Oberfläche des Elements mittels einer
Plasmabehandlung, Einbringen der ersten Komponente in die erzeugten Kavitäten.

24. Verfahren gemäß Anspruch 23, wobei das Element (2, 3) ein Stülpschlauch (3) und/oder ein Endoskopschaft (3) und/oder eine Endoskopschafthülle ist.

25. Verfahren gemäß einem der Ansprüche 23 oder 24, wobei die erste Komponente wenigstens an einem Teil einer Außenfläche des Stülpschlauchs (3) vorgesehen ist.

26. Verfahren gemäß einem der Ansprüche 23 bis 25, wobei das erste Element aus Silikonkautschuk ausgebildet ist.

27. Verfahren gemäß einem der Ansprüche 23 bis 26, wobei die erste und zweite Komponente aus einem körperverträglichen und/oder bioabbaubaren Material ausgebildet sind.

28. Verfahren gemäß einem der Ansprüche 23 bis 27, **dadurch gekennzeichnet, dass** die erste Komponente ein Polysaccharid ist.

29. Verfahren gemäß einem der Ansprüche 23 bis 27, dass die erste Komponente aus Stärke, Glykogen, Cellulose, rekombinanter Cellulose, Agar-Agar oder einem Alginat mit Soja-Protein-Zusatz und/oder Molke-Protein-Zusatz besteht.

30. Verfahren gemäß einem der Ansprüche 23 bis 27, **dadurch gekennzeichnet, dass** die erste Komponente ein Peptid ist.

31. Verfahren gemäß Anspruch 30, **dadurch gekennzeichnet, dass** die erste Komponente aus Kollagen, Gelatine oder rekombinanter Gelatine besteht.

32. Verfahren gemäß einem der Ansprüche 23 bis 31, **dadurch gekennzeichnet, dass** die erste Komponente ein Kunststoff ist.

33. Verfahren gemäß einem der Ansprüche 23 bis 32, **dadurch gekennzeichnet, dass** die zweite Komponente Öl, Wasser, eine Öl-Wasser-Emulsion oder Körperflüssigkeit ist.

34. Verfahren gemäß einem der Ansprüche 23 bis 32, **dadurch gekennzeichnet, dass** die zweite Komponente Ethanol, Glykol, Propanol oder Glycerin ist.

## Claims

1. A medico-technical device comprising at least one element (2, 3) provided with a first component which develops lubrication properties only when a second component is added, **characterized in that** the first component is accommodated in cavities, formed in the surface of the elements (2, 3) wherein the cavities are generated by means of a plasma treatment.

2. A medico-technical device according to claim 1, wherein the element (2, 3) is an everting tube (3) and/or an endoscope shaft (2) and/or and endoscope shaft cover.

3. A medico-technical device according to claim 1 or 2, **characterized in that** the first component is in solid state before the second component is supplied.

4. A medico-technical device according to one of claims 1 to 3, **characterized in that** the first component covers at least partially an outer and/or inner surface of the element (2, 3).

5. A medico-technical device according to one of claims 1 to 4, **characterized in that** the first component has a high bonding energy with respect to the element.

6. A medico-technical device according to one of claims 1 to 5, **characterized in that** the first component has a high flexural elasticity with respect to the element.

7. A medico-technical device according to one of claims 1 to 6, **characterized in that** the element (3) is made from silicon rubber.

8. A medico-technical device according to one of claims 1 to 7, **characterized in that** the first and second component is made from a body compatible and/or biodegradable material.

9. A medico-technical device according to one of claims 1 to 8, **characterized in that** the first component is a polysaccharide.

10. A medico-technical device according to one of claims 1 to 8, **characterized in that** the first component is made from starch, glycogen, cellulose, recombinant cellulose, agar-agar, or an alginate with soybean protein additive and/or whey protein additive.

11. A medico-technical device according to one of claims 1 to 8, **characterized in that** the first component is a peptide.

12. A medico-technical device according to claim 11, **characterized in that** the first component is made from collagen, gelatine or recombinant gelatine.

13. A medico-technical device according to one of claims 1 to 8, **characterized in that** the first component is a synthetic resin.

14. A medico-technical device according to one of claims 1 to 13, **characterized in that** the second component is oil, water, an oil-water emulsion or body fluid.

15. A medico-technical device according to one of claims 1 to 13, **characterized in that** the second component is ethanol, glycol, propanol or glycerine.

16. A medico-technical device according to one of claims 1 to 15, **characterized in that** the medico-technical device is an endoscope (1) having an endoscope shaft (2) and an everting tube (3) at leat patially surrounding said endoscope shaft (2).

17. A medico-technical device according to claim 16, **characterized in that** the second component is applied at a distal eversion (3a) and/or a proximal eversion (3b) of the everting tube (3).

18. A medico-technical device according to one of claims 16 or 17, **characterized in that** the second component is applied between the endoscope shaft (2) and the everting tube (3).

19. A medico-technical device according to one of claims 16 to 18, **characterized in that** the second component is applied between the everted surfaces of said everting tube (3).

20. A medico-technical device according to one of claims 16 to 19, **characterized in that** the second component is applied to a surface of said everting tube facing away from said endoscope shaft (2).

21. A medico-technical device according to one of claims 16 to 20, **characterized in that** the second component is supplied via a channel (6a) formed in said endoscope shaft (2).

22. A medico-technical device according to one of claims 16 to 21, further **characterized in that** an external supply chamber (5, 6) is provided in which the second component is stored and from which the second component is supplied.

23. Method for providing at least one element (2, 3) of a medico-technical device (1) with a first component, which develops its lubrication properties only when being activated by a second component, **characterized by** forming of cavities at the surface of said element by plasma treatment, applying of said first component into said formed cavities.

24. Method according to claim 23, wherein said element (2, 3) is an everting tube (3) and/or an endoscope shaft (3) and/or and endoscope shaft cover.

25. Method according to one of claims 23 or 24, **characterized in that** the first component is provided at at least a part of an outer surface of said everting tube (3).

26. Method according to one of claims 23 to 25, **characterized in that** the first component is made from silicone rubber.

27. Method according to one of claims 23 to 26, **characterized in that** the first and second component is made from a body compatible and/or biodegradable material.

28. Method according to one of claims 23 to 27, **characterized in that** the first component is a polysaccharide.

29. Method according to one of claims 23 to 27, **characterized in that** the first component is made from starch, glycogen, cellulose, recombinant cellulose, agar-agar, or an alginate with soybean protein additive and/or whey protein additive.

30. Method according to one of claims 23 to 27, **characterized in that** the first component is a peptide.

31. Method according to claim 30, **characterized in that** the first component is made from collagen, gelatine, or recombinant gelatine.

32. Method according to one of claims 23 to 31, **characterized in that** the first component is a synthetic resin.

33. Method according to one of claims 23 to 32, **characterized in that** the second component is oil, water, an oil-water emulsion or body fluid.

34. Method according to one of claims 23 to 32, **characterized in that** the second component is ethanol, glycol, propanol or glycerine.

## Revendications

1. Dispositif de technique médicale (1), comportant au moins un élément (2, 3) qui est muni d'un premier composant, qui déploie ses propriétés de lubrification uniquement lors de l'admission d'un deuxième composant,
**caractérisé en ce que** le premier composant est logé dans des cavités qui sont ménagées dans la surface de l'élément (2, 3) et lesdites cavités sont réalisées au moyen d'un traitement par plasma.

2. Dispositif de technique médicale selon la revendication 1, dans lequel l'élément (2, 3) est un tuyau flexible à retournement (3) et/ou une tige d'endoscope (2) et/ou une gaine pour tige d'endoscope.

3. Dispositif de technique médicale selon la revendication 1 ou 2, **caractérisé en ce que** le premier composant est disponible à l'état solide avant l'acheminement avec le deuxième composant.

4. Dispositif de technique médicale selon l'une quelconque des revendications 1 à 3, **caractérisé en ce que** le premier composant recouvre au moins en partie une surface extérieure et/ou surface intérieure de l'élément (2, 3).

5. Dispositif de technique médicale selon l'une quelconque des revendications 1 à 4, **caractérisé en ce que** le premier composant possède une énergie de liaison élevée par rapport à l'élément.

6. Dispositif de technique médicale selon l'une quelconque des revendications 1 à 5, **caractérisé en ce que** le premier composant possède une élasticité de flexion élevée.

7. Dispositif de technique médicale selon l'une quelconque des revendications 1 à 6, **caractérisé en ce que** l'élément (3) est réalisé en caoutchouc siliconé.

8. Dispositif de technique médicale selon l'une quelconque des revendications 1 à 7, **caractérisé en ce que** le premier et le deuxième composant sont réalisés dans un matériau biocompatible et/ou biodégradable.

9. Dispositif de technique médicale selon l'une quelconque des revendications 1 à 8, **caractérisé en ce que** le premier composant est un polysaccharide.

10. Dispositif de technique médicale selon l'une quelconque des revendications 1 à 8, **caractérisé en ce que** le premier composant est constitué d'amidon, de glycogène, de cellulose, de cellulose recombinante, d'agar-agar ou d'un alginate avec un additif de protéine de soja et/ou un additif de protéine de petit-lait.

11. Dispositif de technique médicale selon l'une quelconque des revendications 1 à 8, **caractérisé en ce que** le premier composant est un peptide.

12. Dispositif de technique médicale selon la revendication 11, **caractérisé en ce que** le premier composant est constitué de collagène, de gélatine ou de gélatine recombinante.

13. Dispositif de technique médicale selon l'une quelconque des revendications 1 à 8, **caractérisé en ce que** le premier composant est une matière plastique.

14. Dispositif de technique médicale selon l'une quelconque des revendications 1 à 13, **caractérisé en ce que** le deuxième composant est une huile, de l'eau, une émulsion d'huile et d'eau ou un liquide corporel.

15. Dispositif de technique médicale selon l'une quelconque des revendications 1 à 13, **caractérisé en ce que** le deuxième composant est de l'éthanol, du glycol, du propanol ou de la glycérine.

16. Dispositif de technique médicale selon l'une quelconque des revendications 1 à 15, **caractérisé en ce que** le dispositif de technique médicale est un endoscope (1) comportant une tige d'endoscope (2) et un tuyau flexible à retournement (3) entourant au moins partiellement la tige d'endoscope (2).

17. Dispositif de technique médicale selon la revendication 16, **caractérisé en ce que** le deuxième composant est déposé au niveau d'une zone retournée avant (3a) et/ou d'une zone retournée arrière (3b) du tuyau flexible à retournement (3).

18. Dispositif de technique médicale selon la revendication 16 ou 17, **caractérisé en ce que** le deuxième composant est déposé entre la tige d'endoscope (2) et le tuyau flexible à retournement (3).

19. Dispositif de technique médicale selon l'une quelconque des revendications 16 à 18, **caractérisé en ce que** le deuxième composant est déposé entre les surfaces retournées du tuyau flexible à retournement (3).

20. Dispositif de technique médicale selon l'une quelconque des revendications 16 à 19, **caractérisé en ce que** le deuxième composant est déposé sur une surface du tuyau flexible à retournement, opposée à la tige d'endoscope (2).

21. Dispositif de technique médicale selon l'une quelconque des revendications 16 à 20, **caractérisé en ce que** le deuxième composant est acheminé via un conduit (6a) réalisé dans la tige d'endoscope (2).

22. Dispositif de technique médicale selon l'une quelconque des revendications 16 à 21, **caractérisé en ce qu'**il comporte, en outre, une chambre d'alimentation (5, 6) extérieure, dans laquelle est stocké le deuxième composant et à partir de laquelle le deuxième composant est acheminé.

23. Procédé pour pourvoir au moins un élément (2, 3) d'un dispositif de technique médicale (1) d'un premier composant qui déploie ses propriétés de lubrification uniquement lors de l'admission d'un deuxième composant,
**caractérisé par** la réalisation de cavités sur la surface de l'élément au moyen d'un traitement par plasma, l'introduction du premier composant dans les cavités réalisées.

24. Procédé selon la revendication 23, dans lequel l'élément (2, 3) est un tuyau flexible à retournement (3) et/ou une tige d'endoscope (2) et/ou une gaine pour tige d'endoscope.

25. Procédé selon la revendication 23 ou 24, dans lequel le premier composant est prévu au moins sur une partie d'une surface extérieure du tuyau flexible à retournement (3).

26. Procédé selon l'une quelconque des revendications 23 à 25, dans lequel le premier élément est réalisé en caoutchouc siliconé.

27. Procédé selon l'une quelconque des revendications 23 à 26, dans lequel le premier et le deuxième composant sont réalisés dans un matériau biocompatible et/ ou biodégradable.

28. Procédé selon l'une quelconque des revendications 23 à 27, dans lequel le premier composant est un polysaccharide.

29. Procédé selon l'une quelconque des revendications 23 à 27, dans lequel le premier composant est constitué d'amidon, de glycogène, de cellulose, de cellulose recombinante, d'agar-agar ou d'un alginate avec un additif de protéine de soja et/ou un additif de protéine de petit-lait.

30. Procédé selon l'une quelconque des revendications 23 à 27, dans lequel le premier composant est un peptide.

31. Procédé selon la revendication 30, dans lequel le premier composant est constitué de collagène, de gélatine ou de gélatine recombinante.

32. Procédé selon l'une quelconque des revendications 23 à 31, dans lequel le premier composant est une matière plastique.

33. Procédé selon l'une quelconque des revendications 23 à 32, dans lequel le deuxième composant est une huile, de l'eau, une émulsion d'huile et d'eau ou un liquide corporel.

34. Procédé selon l'une quelconque des revendications 23 à 32, dans lequel le deuxième composant est de l'éthanol, du glycol, du propanol ou de la glycérine.
